# EUROPEAN PATENT SPECIFICATION

(11) **EP 2 018 564 B1**
(45) Date of publication and mention of the grant of the patent: **12.05.2010**
(21) Application number: 07729279.5
(22) Date of filing: 18.05.2007
(51) Int. Cl.: G01N 33/569, C12Q 1/34

(54) **DETECTION METHOD FOR INFLUENZA VIRUSES**
NACHWEISVERFAHREN FÜR GRIPPEVIREN
PROCÉDÉ DE DÉTECTION DES VIRUS DE LA GRIPPE

(30) Priority: 18.05.2006 EP 06114170; 23.08.2006 US 839415 P; 23.08.2006 EP 06119398
(43) Date of publication of application: 28.01.2009
(73) Proprietor: Veterinärmedizinische Universität Wien, 1210 Vienna (AT)
(72) Inventor: BOVIN, Nikolay Vladimirovich, Moscow, 117437 (RU); LUBAVINA, Irina Alexandrovna, Moscow, 125040 (RU); LEISER, Robert-Matthias, 42697 Solingen (DE)
(74) Representative: von Kreisler Selting Werner
(86) International application number: PCT/EP2007/054835
(87) International publication number: WO 2007/135099

(56) References cited:
- US-A- 4 355 102
- US-A1- 2003 129 618
- US-B1- 6 503 745
- NOYOLA DANIEL E ET AL: "Comparison of a new neuraminidase detection assay with an enzyme immunoassay, immunofluorescence, and culture for rapid detection of influenza A and B viruses in nasal wash specimens" JOURNAL OF CLINICAL MICROBIOLOGY, vol. 38, no. 3, March 2000 (2000-03), pages 1161-1165, XP002416855

## Description

The present invention relates to a method of rapid detection of influenza viruses and/or virus particles comprising a hemagglutinin and a neuraminidase component, said method comprising binding the viruses and/or virus particles to a support by a specific binding molecule, which binds to the hemagglutinin component of the viruses and/or virus particles and detecting the bound viruses and/or virus particles by reacting the neuraminidase component with its enzyme substrate resulting in a detectable signal. It further relates to a detection system for the rapid detection of influenza viruses and/or virus particles employing the method according to the invention and the use of the detection system according to the invention.

All avian influenza (AI) viruses are type A influenza virus in the virus family of Orthomyxoviridae and all known strains of influenza A virus infect birds. Influenza virus type A is subdivided into subtypes based on hemagglutinin (H) and neuraminidase (N) protein spikes from the central virus core. There are 16 H types and up to 9 N subtypes, yielding a potential for 144 different H and N combinations.

Avian influenza (also known as bird flu, avian flu, influenza virus A flu, type A flu, or genus A flu) is a flu caused by a type of influenza virus that is hosted by birds, but may infect several species of mammals.

An influenza pandemic is a large scale epidemic of the influenza virus, such as the 1918 Spanish flu. The World Health Organization (WHO) warns that there is a substantial risk of an influenza pandemic within the next few years. One of the strongest candidates is the A (H15N1) subtype of avian influenza.

H5N1 is a type of avian influenza virus (bird flu virus) that has mutated through antigenic drift into dozens of highly pathogenic variants, but all currently belonging to genotype Z of avian influenza virus H5N1. Genotype Z emerged through reassortment in 2002 from earlier highly pathogenic genotypes of H5N1 that first appeared in China in 1996 in birds and in Hong Kong in 1997 in humans. The H5N1 viruses from human infections and the closely related avian viruses isolated in 2004 and 2005 belong to a single genotype, often referred to as genotype Z.

The avian influenza subtypes that have been confirmed in humans, ordered by the number of known human deaths, are: H1N1 caused Spanish flu, H2N2 caused Asian Flu, H3N2 caused Hong Kong Flu, H5N1, H7N7, H9N2, H7N2, H7N3, H10N7.

To be able to respond quickly in case a mutated and virulent new influenza strain capable of human to human transmission emerges requires a quick and reliable test method to determine whether an animal or a person is infected with the virus. Current laboratory methods for the detection of viruses from environmental, animal, and patients' samples are laborious and time-consuming.

US-B-6,503,745 discloses cyclopentane and cyclopentene compounds provided along with the use in a method for detecting influenza virus. The virus is captured using a fetuin coated-surface and detection is performed with a labelled compound which binds to neuraminidase.

D.E. Noyola et al. report in Journal of Clinical Microbiology, 2000, p. 1161 - 1165 about beads coated with fetuin. The fetuin also acts as a substrate for neuraminidase, and detection is carried out using agglutination.

US-A-2003/0129618 discloses methods and compositions for the detection of analytes using a fluorescence that occurs in polymer material in response to selective binding of analytes to the polymeric materials. In particular, the present invention allows for the fluorescent detection of membrane modifying reactions and analytes responsible for such modifications and for the screening of the action inhibitors.

US-B-6,503,745 discloses cyclopentane and cyclopentene compounds and the use in a method for detecting influenza virus.

All known and accessible rapid on-site approaches are based either on the use of antibodies to viral epitopes or on simple neuraminidase (NA) tests. The main disadvantage of antibody approaches based on a lateral flow technique or other assay types is the instability of antibody reagents. Furthermore, the majority of immunological assays available so far show a specificity for influenza A, but not H5N1. Additionally, the use of antibodies unavoidably leads to an increase of assay costs.
The main disadvantage of NA tests specific for influenza viruses is the necessary expensive substrate reagent used in the state of the art, which should be partially methylated.

The present invention solves these problems by providing a method for the rapid detection of influenza viruses and/or virus particles, a detection system employing said method as well as the use of said detection system as defined in the claims.

The following abbreviations will be used in the specification: 3'SLN = Neu5Aca2-3Galβ1-4GlcNAc HA = hemagglutinin; HAR = reaction of hemagglutination; LOD = limit of detection; NA = neuraminidase; TCID = tissue culture infectious dose; TN buffer = 0.02 M tris-HCl (pH 7.2) with 0.1 M -of NaCl.

According to the invention a method of rapid detection of influenza virus and/or virus particles is employed, the method comprising the steps of:
a) binding the viruses and/or virus particles to a support containing at least one type of carbohydrate receptor selected from the group consisting of natural or synthetic oligosaccharide, which is conjugated to, or situated in composition with glycoproteins like glycophorin, a1-acid glycoprotein, a2-macroglobulin, ovomucoid, and combinations thereof which carbohydrate receptor binds to the hemagglutinin component of the viruses and/or virus particles;
b) reacting the neuraminidase component of the bound viruses and/or virus particles with its labelled enzyme substrate, causing the generation of a detectable signal; and
c) detecting the signal generated in step b).

In particular, influenza viruses and/or virus particles comprising all known Avian Influenza (AI) sub-types are detected.

In another embodiment of the invention the influenza viruses and/or virus particles comprise a certain sub-type or group of sub-types. The method is suitable to detect influenza viruses and/or virus particles comprising a highly pathogenic variant.

The invention can be performed in particular with a support which is a chromatographic paper or membrane. Such materials are well-known to the skilled person. According to the invention it is possible to covalently attach or physically adsorb the carbohydrate receptor. as referred hereinabove, to the support.

The invention employs in particular a carbohydrate receptor containing the α2-3Gal motive. According to one embodiment of the invention (dot-blot approach) the binding of the viruses and/or virus particles is effected by loading a virus and/or virus particles containing sample to the support as a spot. According to the invention binding of the viruses and/or virus particles is effected by soaking a virus and/or virus particles containing sample along said support in form of so-called lateral-flow approach. In another embodiment of the invention a labeled enzyme substrate of the neuraminidase component is precipitated on the place of the bond virus and/or virus particles.

In yet another embodiment of the present invention the enzyme substrate is labeled with a chromogenic group and the reaction with a neuraminidase induces a color change of said enzyme substrate. As enzyme substrates may be employed among others, the following chromogenic derivatives of N-acetyl neuraminic acid, in particular 5-bromo-4-chloro-3-indolyl-α-N-actyl neuraminic acid. Alternatively, the reaction with the neuraminidase induces a specific fluorescence signal when employing suitable fluorescent molecules as a label.

The method of the invention can be advantageously performed with a detection system for the rapid detection of influenza viruses and/or virus particles according to the invention. The system comprises a detection system for the rapid detection of influenza viruses and/or virus particles using the method of the invention, said system comprising:
a) a support containing at least one type of a carbohydrate receptor selected from the group consisting of natural or synthetic oligosaccharide, which is conjugated to, or situated in composition with glycoproteins like glycophorin, a1-acid glycoprotein, a2-macroglobulin, ovomucoid, and combinations thereof which carbohydrate receptor binds to the hemagglutinin component of the viruses and/or virus particles;
b) a labelled enzyme substrate which reacts with the neuraminidase component, thereby generating a detectable signal.

In particular, the support is enclosed in a plastic holder with a window for reading the test result with a sample and a positive control. The support comprises in particular at least two different amounts of said specific binders for semi-quantitative estimation of the virus content in the sample. In another embodiment the support comprises at least two specific receptors of different sub-type specificity for simultaneous detection of viruses belonging to different sub-types. The detection system of the invention serves as sample container and transport unit for confirmatory tests like reversed transcription polymerase chain reaction after revealing the presence of the virus and/or virus particles of sought specificity.

According to the invention also the use of a detection system is claimed for the detecting of influenza viruses and/or virus particles in samples of animals and/or humans like swabs, faeces and blood in environmental samples and/or as an early warning system as of emerging high pathogenic virus sub-types.

Hemagglutinin (HA) and neuraminidase (NA) are present on the surface of an influenza virus. HA induces virus binding to sialyl-containing cell receptors, while NA promotes virus access to target cells and facilitates virus release from infected cells and natural inhibitors [Matrosovich, M.N., Matrosovich, T.Y., Gray, T., Roberts, N.A., Klenk, H.D. 2004: J. Virol.. 78(22) 12665-7], i.e. HA is receptor-binding whereas NA is receptor-destroying. An important condition for an efficient virus replication is the concerted action of HA and NA, and for different species the relation of these two activities is different. Influenza viruses isolated from birds display some distinctive features. All avian influenza viruses possess HA, which has the highest affinity for Neu5Acα2-3Gal-terminated carbohydrate chains. Furthermore, their NA activity is higher compared to viruses of other origins.

In the detection method and detection system according to the present invention, these properties of both virion components, HA and NA, of avian viruses are used. An influenza virus will be bound to a specific binding molecule, preferably to *a substance* that contains sialylated carbohydrates and is coupled to a membrane, followed by reaction with, preferably digestion of a neuraminidase substrate causing dying of the virus-containing zone on the membrane. The flu virus detection method and system according to the present invention display a variety of advantages compared to test systems of the state of the art: using two components, HA and NA, increases the specificity of the assay and allows the construction of detection systems with chosen specificities. At the same time and in contrast to detection systems based on immunological methods, there is no need for an additional marker enzyme for revealing the positive detection, because the viral neuraminidase itself is used for this purpose.

The different variants of the thoroughly chosen types of carbohydrate receptor molecules will collect all influenza viruses independent of type and subtype, and are capable of simultaneous specific detection of highly pathogenic variants like H5N1.

The result of the detection method according to the invention can be obtained without the use of any sophisticated devices.

The method according to the invention allows the detection of influenza viruses within 0.1 to 2 hours.

The invention will be explained further by way of the following, non-limiting examples.

### Examples

### Example 1:

### Synthesis of a polyacrylamide conjugate of 3'SLN trisaccharide for preparation of the specific carbohydrate receptor molecule

A solution of 1.7 mg (10 µmol) polyacrylic acid completely activated with N-hydroxysuccinimide, MW ∼1000 kDa, in 200 µl DMSO, and 4 µl diisopropylethylamine were added to a solution of 1.46 mg (2 µmol) 3'SLN-O(CH₂)₃NH₂ in 200 µl DMSO, the mixture was kept at 37°C for 48 h, 40 µl of 25% solution aqueous ammonia or 40 µl ethanolamine were added and the solution was kept for 18 h at room temperature followed by gel-permeation chromatography on sepharose LH, yield ∼ 90%.

### Example 2:

### Detection of influenza viruses in allantoic fluid by using the method and test system according to the invention

### Example-Kit:

1. Test-strip
2. Probe buffer Buffer #1
3. Contrasting buffer Buffer #2
4. Washing buffer A Buffer #3
5. Washing buffer B Buffer #4
6. Dying buffer Buffer #5
7. Double strip (2 rows with 8 wells) or 96-well plate
8. Scissors or scalpel
9. Pincers
10. Plate for strip washing
11. Development vials
12. Thermostat (or its surrogate)
13. Camera for chromatography procedure (or a lid, which is used for covering the 96-well plate with strips).

### Materials

Strips: nitrocellulose, absorbent, sample pad obtained from Whatman, USA.
1. Specific reagent (fetuin, Sigma) was isolated from fresh chicken eggs.
2. Buffer #1: 0.2 M tris-HCl (pH 7.2) with 3 M of NaCl and 0.5% of Tween-20
3. Buffer #2: 0.02 M tris-HCl (pH 7.2) with 0.3 M of NaCl and 0.05% of Tween-20
4. Buffer #3: 0.02 M tris-HCl (pH 7.2) with 0.1 M of NaCl and 0.05% of Tween-20
5. Buffer #4: 0.02 M tris-HCl (pH 7.2) with 0.1 M of NaCl (TN buffer)
6. Buffer #5: 4 mM solution of Neu-X in TN buffer with 0.01 M CaCl₂ TN buffer can be substituted with another fitting buffer, e.g. phosphate buffered saline or saline.

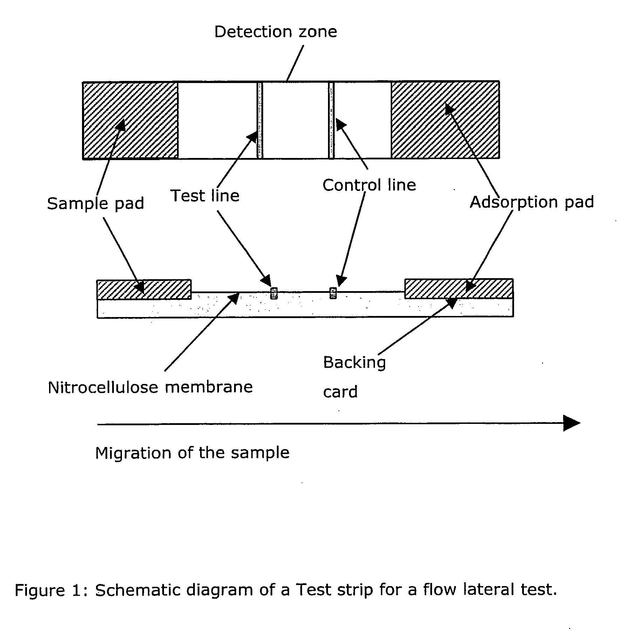

The strip (Fig. 1) is divided into three zones: soaking, detection and absorbing zones. A line of the carbohydrate receptor molecule (test line) as well as a control line are located in the detection zone. The strips are assembled from the membrane, the absorbent and the sample pads. The carbohydrate receptor molecule (1 microliter of 1 mg/mL solution) is loaded on the detection zone, after that the strip is dried and washed. Then the strip is stored in a hermetically closed flask at 18-25 °C.

### Detection

**1. Sample preparation.** 1/10 part (v/v) Buffer #1 is added to the probe directly before analysis and shaken well. This is probe #1 (P #1).
**2. Detection procedure involves several stages (Table 1).**

**At the first stage** the sample pad of the strip is placed into 80-100 µl of P #1. Upon action of capillary forces liquid from the sample is soaked up and enters the absorbing zone via the detection zone where virus particles bind to the carbohydrate receptor molecule. The duration of this procedure depends on the sample viscosity, but should not exceed 15 min. Buffer #2 is added to the same well of the plate (alternatively it is possible to transfer the strip to another well filled with Buffer #2). After 10 min the strip is taken out of the well.

**At the second stage,** the soaking part of the strip is cut (with scissors). After careful removal (e.g. with the help of pincers) of the absorbing zone of the strip part, the test zone is washed with Buffer #3 and then Buffer #4. Subsequently the absorbing zone is cut too.

**The third stage.** The rest of the test zone is placed into the flask with Buffer#5. The flask is tightly closed and allowed to stand in the dark at 37 - 40°C. Presence of an influenza virus in the sample is detected as a dark-blue dying of the carbohydrate receptor molecule zone. Duration of this step depends on the virus concentration in the sample and can take from 20 min till 60 min before a virus can be detected by hemagglutination reaction (HAR titer about 2). As a rule these samples have TCID/ml value of about 10⁵. For probes with 10 and 100-fold less virus content (10⁴-10³ TCID/ml), dying may appear after several hours. After this procedure the strip is taken out of the solution followed by visual evaluation of the reaction result.

**The Control line** of neuraminidase in the detection zone is used for evaluation of the assay functionality. Dark-blue dying of the *control zone* must take place.

### Results are interpreted in the following way:

1) If an evenly dyed line is observed, it is considered positive, i.e. the probe contains the virus; color intensity should be comparable with intensity of the control line.
2) If a weakly dyed line is observed, the sample contains the virus in a concentration that is insufficient for unambiguous detection, and the strip exposition in Buffer #5 should be prolonged overnight or the test may be repeated once more.
3) Absence of the colored line means that there is no virus in the sample or its concentration is less then the minimal detection level.
4) Absence of the colored line in the positive control line either means that the experiment was performed incorrectly or that the NA in the control line has been destroyed; in that case it is recommended to re-test the specimen using a new test kit.

The data on the test sensitivity are given in Table 2.

**Table 1: Scheme of the detection method according to the invention**

| STEP | PROCEDURE | FIGURE | CONDITION |
|---|---|---|---|
| 1 | Pre-testing preparation | | |
| 2 | Soaking/ chromatography | 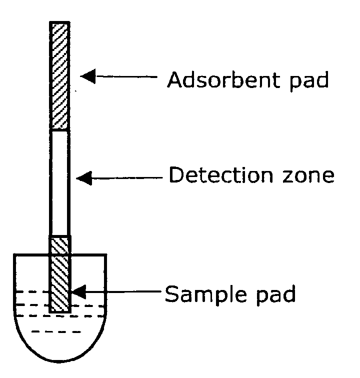 | Sample pad of the strip is placed vertically to specimen container for 10 min at room temperature |
| 3 | Washing | 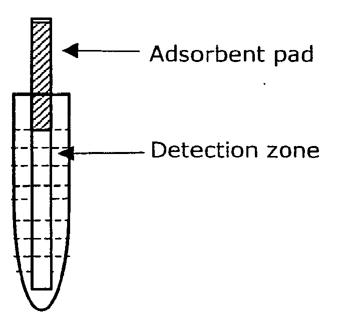 | After sample pad removal the remaining part of the strip is placed into the washing solution |
| 4 | Development in the dark | 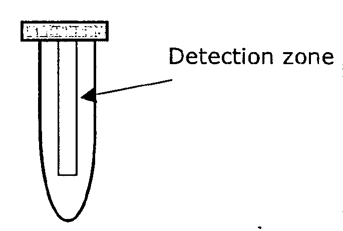 | 20 min - 2 h in the dark at 40°C in neuraminidase substrate solution |
| 5 | Interpretation of the results | 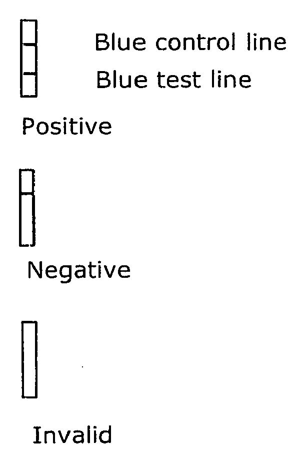 | The results are evaluated visually. In case of the positive result (sample contains virus in conc. more than detection limit) two lines should be observed. Dying intensity of the test line should be comparable with that of the control line. In case of the negative result only the control line is observed. It is necessary to repeat the test when the control line can not be observed. |
| | | | |

### Example 3:

### Detection of influenza viruses in chicken faeces using the detection system according to the invention

In this example the same materials and methods were used as in example 2.
1. Serial dilutions of virus-containing probes in suspension of healthy chicken faeces (2 g of dry substance per 10 ml of buffer) were performed. It has been demonstrated that faeces do not affect sensitivity and specificity of influenza virus detection with the test.
2. To fresh chicken faeces 1/10 part (v/v) of A/duck/Alberta/76 virus (HAR-titer 1:10) was added and the mixture was homogenized. After addition of Buffer #2 to the homogenate (1:1 v/v), an aliquot of the resulting suspension was taken for use in the assay. Dark-blue dying in the test zone appeared after 2 h. No remarkable influence on the test results was observed when chicken faeces were added to the samples from table 1 before starting the test procedure.

### Example 4:

### Detection of influenza viruses in lung tissue of dead chicken using the detection system according to the invention

In this example the same materials and methods were used as in example 2.

Lung tissue from chicken that died of infection with influenza virus H5N1 has been extracted with silin buffer analyzed (collected: Crimea/January 2006). It could be shown that influenza virus can be reliably detected in the lung of the chicken body analyzed.

**Table 2: Limit of detection (LOD) of influenza avian viruses in virus-containing samples as probed with the test system according to the invention**

| **N°** | **VIRUS** | **LOD for 2 h** | |
|---|---|---|---|
| | | **TCID /ml** | **HAR-titer/sample** |
| 1 | A/duck/Alberta/35/76 (H1N1) | 10⁵ | 4 |
| 2 | A/duck/France/146/82 (H1N1) | 2 x 10⁴ | 10⁻² |
| 3 | A/pintail/Primorie/695/76 (H2N3) | 2 x 10⁷ | 10 |
| 4 | A/gull/Astrakhan/165/86 (H6N5) | 10³ | 10⁻¹ |
| 5 | A/FPV/Rostock/34 (H7N1) | 10⁴ | 1 |
| 6 | A/mallard/NT/12/02 (H7N3) | 5 x 10⁴ | 5 x 10⁻¹ |
| 7 | A/mallard/Primorie/3/82 (H9N2) | 10⁶ | 10⁻¹ |
| 8 | A/Hongkong/1073/99 (H9N2) | 10⁵ | 1 |
| 9 | A/mallard/Guriev/244/82 (H14N6) | 5 x 10⁶ | 10⁻¹ |
| 10 | A/mallard/PA/10218 (H5N2) | Not determined | 10⁻² |
| 11 | A/duck/Postdam/1402/6/86 (H5N2) | 5 x 10² | 5 x 10⁻¹ |
| 12 | A/NIBRG-14 (H5N1)^{a} | 5 x 10³ | 5 |
| 13 | A/chicken/Kurgan/5/2005 (H5N1) 2 passages in MDCK | 10⁴ 10⁴ | <1 |
| 14 | A/duck/Kurgan/8/2005(H5N1) 2 passages in MDCK | 10⁶ | 10⁻¹ |

| | | | |
|---|---|---|---|
| ^{a} - This is vaccine strain, having HA and NA genes from the A/Vietnam/1194/04 pathogen virus and all inner protein genes from A/Puerto Rico/2/34. Viruses were obtained from the Institute of Poliomyelitis and Viral Encephalitis (Moscow Region) and Institute of Influenza (St. Petersburg). | | | |

## Claims

1. A method of rapid detection of influenza viruses and/or virus particles comprising a hemagglutinin and a neuraminidase component, said method comprising the steps of:
a) binding the viruses and/or virus particles to a support containing at least one type of carbohydrate receptor selected from the group consisting of natural or synthetic oligosaccharide, which is conjugated to, or situated in composition with glycoproteins which carbohydrate receptor binds to the hemagglutinin component of the viruses and/or virus particles;
b) reacting the neuraminidase component of the bound viruses and/or virus particles with its labelled enzyme substrate, causing the generation of a detectable signal; and
c) detecting the signal generated in step b).

2. The method of claim 1 wherein the glycoprotein is selected from the group consisting of glycophorin, a1-acid glycoprotein, a2-macroglobulin, ovomucoid, and combinations thereof

3. The method according to claim 1 or 2 wherein said influenza viruses and/or virus particles comprise a certain subtype or a group of subtypes, in particular all known avian influenza (AI) subtypes.

4. The method according to claim 1, wherein said influenza viruses and/or virus particles comprise a highly pathogenic variant.

5. The method according to any of claims 1 to 4, wherein the support is a chromatographic paper or membrane.

6. The method according to any of claims 1 to 5, wherein the carbohydrate receptor, in particular wherein the carbohydrate receptor contains the alpha2-3Gal motif, is covalently attached or physically adsorbed to the support.

7. The method according to any of claims 1 to 6, wherein said binding of said viruses and/or virus particles is effected by loading a virus and/or virus particles containing sample to said support as a spot, or wherein said binding of said viruses and/or virus particles is effected by soaking a virus and/or virus particles containing sample along said support.

8. The method according to any of claims 1 to 7, wherein said labelled enzyme substrate of said neuraminidase component is precipitated on the place of the bound viruses and/or virus particles.

9. The method according to any of claims 1 to 8, wherein said enzyme substrate is labelled with a chromogenic group and the reaction with the neuraminidase induces a colour change of said enzyme substrate, in particular said enzyme substrate is a chromogenic derivative of N-acetylneuraminic acid, in particular 5-bromo-4-chloro-3-indolyl-α-N-acetylneuraminic acid.

10. The method according to any of claims 1 to 9, wherein the reaction with the neuraminidase induces a specific fluorescence signal.

11. A detection system for the rapid detection of influenza viruses and/or virus particles using a method according to any of claims 1-10, said system comprising:
a) a support containing at least one type of a carbohydrate receptor selected from the group consisting of natural or synthetic oligosaccharide, which is conjugated to, or situated in composition with glycoproteins, which carbohydrate receptor binds to the hemagglutinin component of the viruses and/or virus particles;
b) a labelled enzyme substrate which reacts with the neuraminidase component, thereby generating a detectable signal.

12. The detection system according to claim 11, wherein said support is enclosed in a plastic holder with a window for reading the test results with a sample and a positive control.

13. The detection system according to claim 11 and/or 12, wherein said support comprises at least two different amounts of said specific binders for a semi-quantitative estimation of the virus content in the sample.

14. The detection system according to claim 11 to 13, wherein said support comprises at least two specific receptors of different subtype specificity for a simultaneous detection of viruses belonging to different subtypes.

15. The detection system according to any of claims 11 - 14, wherein the system after revealing the presence of the virus and/or virus particles of sought specificity serves as sample container and transport unit for confirmatory tests like reverse transcription polymerase chain reaction.

16. Use of the detection system according to any of claims 11 to 15 for detecting influenza viruses and/or virus particles in samples of animals and/or humans like swabs, faeces and blood, in environmental samples and/or as an early warning system of emerging highly pathogenic virus subtypes.

## Patentansprüche

1. Verfahren zum schnellen Nachweis von Influenzaviren und/oder Viruspartikeln, die eine Hämagglutinin- und eine Neuraminidasekomponente umfassen, wobei das Verfahren die folgenden Schritte umfasst:
a) Binden der Viren und/oder Viruspartikel an einen Träger, der wenigstens einen Typ von Kohlenhydratrezeptor enthält, welcher aus der Gruppe, die aus natürlichem oder synthetischem Oligosaccharid besteht, ausgewählt ist und an Glycoproteine konjugiert ist oder sich in deren Gesellschaft befindet, wobei der Kohlenhydratrezeptor an die Hämagglutininkomponente der Viren und/oder Viruspartikel bindet;
b) Umsetzen der Neuraminidasekomponente der gebundenen Viren und/oder Viruspartikel mit ihrem markierten Enzymsubstrat, was die Erzeugung eines nachweisbaren Signals bewirkt; und
c) Nachweisen des in Schritt b) erzeugten Signals.

2. Verfahren gemäß Anspruch 1, wobei das Glycoprotein aus der Gruppe ausgewählt ist, die aus Glycophorin, a1-Säure-Glycoprotein, a2-Makroglobulin, Ovomucoid und Kombinationen davon besteht.

3. Verfahren gemäß Anspruch 1 oder 2, wobei die Influenzaviren und/oder Viruspartikel einen bestimmten Subtyp oder eine Gruppe von Subtypen, insbesondere alle bekannten Vogelgrippe(AI)-Subtypen, umfassen.

4. Verfahren gemäß Anspruch 1, wobei die Influenzaviren und/oder Viruspartikel eine hochgradig pathogene Variante umfassen.

5. Verfahren gemäß einem der Ansprüche 1 bis 4, wobei der Träger ein Chromatographiepapier oder eine Chromatographiemembran ist.

6. Verfahren gemäß einem der Ansprüche 1 bis 5, wobei der Kohlenhydratrezeptor, der insbesondere das α2-3gal-Motiv enthält, an dem Träger kovalent gebunden oder physikalisch adsorbiert ist.

7. Verfahren gemäß einem der Ansprüche 1 bis 6, wobei die Bindung der Viren und/oder Viruspartikel **dadurch** bewirkt wird, dass man eine Virus und/oder Viruspartikel enthaltende Probe als Fleck auf den Träger gibt, oder wobei die Bindung der Viren und/oder Viruspartikel **dadurch** bewirkt wird, dass man eine Virus und/oder Viruspartikel enthaltende Probe entlang dem Träger aufsaugen lässt.

8. Verfahren gemäß einem der Ansprüche 1 bis 7, wobei das markierte Enzymsubstrat der Neuraminidase-Komponente auf die Stelle der gebundenen Viren und/oder Viruspartikel ausgefällt wird.

9. Verfahren gemäß einem der Ansprüche 1 bis 8, wobei das Enzymsubstrat mit einer chromogenen Gruppe markiert ist und die Reaktion mit der Neuraminidase eine Farbänderung des Enzymsubstrats induziert, wobei das Enzymsubstrat insbesondere ein chromogenes Derivat von N-Acetylneuraminsäure, insbesondere 5-Brom-4-chlor-3-indolyl-α-N-acetylneuraminsäure, ist.

10. Verfahren gemäß einem der Ansprüche 1 bis 9, wobei die Reaktion mit der Neuraminidase ein spezifisches Fluoreszenzsignal induziert.

11. Nachweissystem für den schnellen Nachweis von Influenzaviren und/oder Viruspartikeln mit Hilfe eines Verfahrens gemäß einem der Ansprüche 1 bis 10, wobei das System Folgendes umfasst:
a) einen Träger, der wenigstens einen Typ von Kohlenhydratrezeptor enthält, welcher aus der Gruppe, die aus natürlichem oder synthetischem Oligosaccharid besteht, ausgewählt ist und an Glycoproteine konjugiert ist oder sich in deren Gesellschaft befindet, wobei der Kohlenhydratrezeptor an die Hämagglutininkomponente der Viren und/oder Viruspartikel bindet;
b) ein markiertes Enzymsubstrat, das mit der Neuraminidasekomponente reagiert, wodurch ein nachweisbares Signal erzeugt wird.

12. Nachweissystem gemäß Anspruch 11, wobei der Träger in einem Kunststoffhalter mit einem Fenster zum Ablesen der Testergebnisse mit einer Probe und einer positiven Kontrolle eingeschlossen ist.

13. Nachweissystem gemäß Anspruch 11 und/oder 12, wobei der Träger wenigstens zwei unterschiedliche Mengen der spezifischen Bindemittel für eine semiquantitative Abschätzung des Virusgehalts in der Probe umfasst.

14. Nachweissystem gemäß Anspruch 11 bis 13, wobei der Träger wenigstens zwei spezifische Rezeptoren mit unterschiedlicher Subtypspezifität für einen gleichzeitigen Nachweis von Viren, die zu verschiedenen Subtypen gehören, umfasst.

15. Nachweissystem gemäß einem der Ansprüche 11 bis 14, wobei das System nach dem Aufdecken der Anwesenheit des Virus und/oder der Viruspartikel der gesuchten Spezifität als Probenbehälter und Transporteinheit für Bestätigungstests, wie RT-PCR (reverse transcription polymerase chain reaction) dient.

16. Verwendung des Nachweissystems gemäß einem der Ansprüche 11 bis 15 zum Nachweisen von Influenzaviren und/oder Viruspartikeln in Proben von Tieren und/oder Menschen, wie Abstrichen, Kot und Blut, in Umweltproben und/oder als Frühwarnsystem für das Auftreten hochgradig pathogener Virussubtypen.

## Revendications

1. Procédé de détection rapide de virus et/ou de particules virales de la grippe comprenant un composant hémagglutinine et un composant neuraminidase, ledit procédé comprenant les étapes de :
a) fixation des virus et/ou des particules virales sur un support contenant au moins un type de récepteur de sucre, choisi dans le groupe formé par les oligosaccharides naturels ou synthétiques, conjugué à ou situé dans une composition contenant des glycoprotéines, ledit récepteur de sucre fixant le composant hémagglutinine des virus et/ou des particules virales ;
b) la réaction du composant neuraminidase des virus et/ou des particules virales fixés avec son substrat enzymatique marqué, provoquant la génération d'un signal détectable ; et
c) la détection du signal généré à l'étape b).

2. Procédé selon la revendication 1, dans lequel la glycoprotéine est choisie dans le groupe formé par la glycophorine, l'a1-glycoprotéine acide, l'a2-macroglobuline, l'ovomucoïde, et leurs combinaisons.

3. Procédé selon la revendication 1 ou 2, dans lequel lesdits virus et/ou particules virales de la grippe comprennent un certain sous-type ou groupe de sous-types, en particulier tous les sous-types connus de la grippe aviaire (GA).

4. Procédé selon la revendication 1, dans lequel lesdits virus et/ou particules virales de la grippe comprennent une variante hautement pathogène.

5. Procédé selon l'une quelconque des revendications 1 à 4, dans lequel le support est une membrane ou un papier chromatographique.

6. Procédé selon l'une quelconque des revendications 1 à 5, dans lequel le récepteur de sucre, en particulier lorsque le récepteur de sucre contient le motif alpha2-3Gal, est lié par liaison covalente à ou physiquement adsorbé sur le support.

7. Procédé selon l'une quelconque des revendications 1 à 6, dans lequel ladite fixation desdits virus et/ou particules virales s'effectue au moyen du dépôt d'un échantillon contenant des virus et/ou des particules virales sur ledit support sous la forme d'une tache, ou dans lequel ladite fixation desdits virus et/ou particules virales s'effectue au moyen de l'imprégnation d'un échantillon contenant des virus et/ou des particules virales le long dudit support.

8. Procédé selon l'une quelconque des revendications 1 à 7, dans lequel ledit substrat enzymatique marqué dudit composant neuraminidase est précipité à l'emplacement de fixation des virus et/ou des particules virales.

9. Procédé selon l'une quelconque des revendications 1 à 8, dans lequel ledit substrat enzymatique est marqué par un groupe chromogène et la réaction avec la neuraminidase induit un changement de couleur dudit substrat enzymatique, en particulier ledit substrat enzymatique est un dérivé chromogène de l'acide N-acétylneuraminique, en particulier l'acide 5-bromo-4-chloro-3-indolyl-α-N-acétylneuraminique.

10. Procédé selon l'une quelconque des revendications 1 à 9, dans lequel la réaction avec la neuraminidase induit un signal de fluorescence spécifique.

11. Système de détection pour la détection rapide de virus et/ou de particules virales de la grippe utilisant un procédé selon l'une quelconque des revendications 1 à 10, ledit système comprenant :
a) un support contenant au moins un type de récepteur de sucre, choisi dans le groupe formé par les oligosaccharides naturels ou synthétiques, conjugué à ou situé dans une composition contenant des glycoprotéines, ledit récepteur de sucre fixant le composant hémagglutinine des virus et/ou des particules virales ;
b) un substrat enzymatique marqué qui réagit avec le composant neuraminidase, générant ainsi un signal détectable.

12. Système de détection selon la revendication 11, dans lequel ledit support est enfermé dans un portoir de matière plastique, présentant un hublot pour la lecture des résultats de l'essai, avec un échantillon et un témoin positif.

13. Système de détection selon les revendications 11 et/ou 12, dans lequel ledit support comprend au moins deux quantités différentes desdits agents de fixation spécifiques pour une estimation semi-quantitative de la teneur en virus de l'échantillon.

14. Système de détection selon les revendications 11 à 13, dans lequel ledit support comprend au moins deux récepteurs spécifiques présentant des spécificités de sous-type différentes, pour une détection simultanée de virus appartenant à différents sous-types.

15. Système de détection selon l'une quelconque des revendications 11 à 14, dans lequel le système, après avoir révélé la présence des virus et/ou des particules virales de la spécificité recherchée, sert de contenant d'échantillon et d'unité de transport pour des essais de confirmation tels qu'une réaction en chaîne par polymérase après transcription inverse.

16. Utilisation du système de détection selon l'une quelconque des revendications 11 à 15 pour la détection de virus et/ou de particules virales de la grippe, dans des échantillons d'animaux et/ou d'êtres humains tels que des frottis, des selles et du sang, dans des échantillons environnementaux et/ou en tant que système d'alerte précoce de l'émergence de sous-types de virus hautement pathogènes.
